# EUROPÄISCHE PATENTANMELDUNG

(11) **EP 2 693 352 A1**
(43) Veröffentlichungstag der Anmeldung: **05.02.2014**
(21) Anmeldenummer: 12178598.4
(22) Anmeldetag: 31.07.2012
(51) Int. Cl.: G06F 19/00

(54) **System zur Übertragung personenbezogener und nicht personenbezogener Daten (Data-Split)**

(71) Anmelder: Monks Vertriebsges. mbH, 81539 München (DE)
(72) Erfinder: Monks, Sean, 81545 München (DE)
(74) Vertreter: Körfer, Thomas

(57) **Zusammenfassung**

Die Erfindung stellt ein Datenübertragungssystem bereit, das personenbezogene und nicht personenbezogene medizinische Daten überträgt, vorzugsweise medizinische, Daten, überträgt, aufweisend ein ein Formularsystem bereitstellendes erstes Kommunikationsgerät, wobei das Formularsystem dazu eingerichtet ist, Falldaten zu erfassen und wenigstens eine personenbezogene Datenkomponente aus den Falldaten zu extrahieren und zu wenigstens einem ersten Speicher zu übertragen, wenigstens eine weitere Datenkomponente aus den Falldaten zu extrahieren und zu wenigstens einem zweiten Speicher zu übertragen, und ein elektronisches Dokument zu generieren und zu einem zweiten Kommunikationsgerät zu senden, und das zweite Kommunikationsgerät, das dazu eingerichtet ist, das elektronische Dokument zu empfangen, und nach Empfang des elektronischen Dokuments hin die wenigstens eine personenbezogene Datenkomponente von dem ersten Speicher und die wenigstens eine weitere Datenkomponente von dem zweiten Speicher abzurufen und mit Daten aus dem elektronischen Dokument zu den Falldaten zusammenzuführen und für die Bearbeitung auf dem zweiten Kommunikationsgerät bereit zu stellen.

## Beschreibung

### Gebiet der Erfindung

Die Erfindung bezieht sich auf ein Computersystem zur automatischen Trennung und gesicherten Übertragung von personenbezogenen und nicht personenbezogenen Patientendaten im medizinischen Bereich. Hierbei werden personenbezogene Patientendaten und nicht personenbezogene Patientendaten von einer Quelle zu einem Empfänger übermittelt. Die Übertragung erfolgt dabei so, dass ein Zusammenführen der personenbezogenen und der nicht personenbezogenen Daten nicht ohne weiteres erfolgen kann.

### Hintergrund der Erfindung

Im Gesundheitssystem ist eine Entwicklung dahingehend zu beobachten, dass Fachärzte sich zunehmend vermehrt in städtischen Gebieten ansiedeln, während in ländlichen Bereichen eine fachärztliche Versorgung teilweise nicht mehr gesichert ist. Ein Grund hierfür ist, dass diese Bereiche für eine Niederlassung weniger attraktiv sind. Auch werden fachärztliche Praxen teilweise vermehrt von mehreren Ärzten betrieben, die jeweils in Teilzeit arbeiten, und daher zu wechselnden und unterschiedlichen Zeiten zur Verfügung stehen.

Für die Patienten stellt sich daher das Problem, dass sie, wenn sie von einem Arzt zu einem Facharzt überwiesen werden, weite Strecken auf sich nehmen müssen, um diesen zu erreichen und die fachspezifische Beratung zu erhalten. Insbesondere alters- oder krankheitsbedingt kann dies für Patienten sehr umständlich und aufwendig sein.

Es stellt sich daher das Problem, wie dieses Konsultationsverfahren vereinfacht werden kann und wie beispielsweise eine bestehende Kommunikationsinfrastruktur für die Konsultation genutzt werden kann, um sowohl dem Patienten einen Facharztbesuch zu ersparen, auf der anderen Seite aber den behandelnden Arzt in die Lage zu versetzen, fachärztlichen Rat einholen zu können. Als Nebeneffekt muss der konsultierende Arzt dann auch das Risiko nicht mehr eingehen, Patienten an einen anderen Arzt zu verlieren.

### Kurze Zusammenfassung der Erfindung

Die Erfindung richtet sich auf die technischen Aspekte mit denen dies zu erreichen ist.

Das der Erfindung zu Grunde liegende Prinzip ist darin zu sehen, dass ein Patient einen Arzt, im Folgenden konsultierende Partei, aufsucht. Die konsultierende Partei erfasst mit Hilfe des Systems und des Verfahrens Falldaten, die personenbezogene und nicht personenbezogene Daten umfassen.

Diese Falldaten werden anschließend durch das erfinderische System an eine konsultierte Partei (z.B. einen Facharzt) übermittelt. Dabei stellt das System und das Verfahren die Informationen so bereit, dass eine Übermittlung der personenbezogenen und der nicht personenbezogenen Daten so erfolgt, dass die konsultierte Partei asynchron, d.h. auch in Abwesenheit der konsultierenden Partei, Zugriff auf die Falldaten erhalten kann, während die Übermittlung zu der konsultierten Partei ebenso in deren Abwesenheit erfolgen kann.

Das System und das Verfahren stellen dabei sicher, dass die konsultierte Partei nur dann Zugriff auf die Daten erhalten kann, wenn sie durch weitere Information dazu autorisiert wird. Dies kann z.B. durch Übertragung und/oder auf Basis eines weiteren Autorisierungsdatensatzes, z.B. eines elektronischen Dokuments, erfolgen.

Die konsultierte Partei ist dann in der Lage, anhand der Falldaten eine Beurteilung abzugeben, weitere Fragen zu dem Fall zu stellen, oder weitere Informationen anzufordern.

Nach Abschluss der Bearbeitung durch die konsultierte Partei werden die bearbeiteten Falldaten dann ebenfalls wieder über das erfindungsgemäße System mit Hilfe des erfindungsgemäßen Verfahrens an die konsultierende Partei übermittelt, der dann die bearbeiteten Falldaten zur Verfügung stehen.

In einem konkreten Beispiel sendet also ein Arzt (Allgemeinmediziner/Hausarzt) als konsultierende Partei Falldaten an einen Facharzt, der nach Bearbeitung der Falldaten, bzw. dem Erstellen einer fachlichen Beratung auf Basis der Falldaten, die bearbeiteten Falldaten an den Arzt zurücksendet. Dieser verwendet dann die bearbeiteten Falldaten für die Patientenberatung und beispielsweise zur Therapieplanung.

Das oben genannte Problem wird also durch ein System und ein Verfahren gelöst, wie es jeweils mit den unabhängigen Ansprüchen beansprucht wird. Weitere Ausgestaltungen der Erfindung sind Gegenstand der abhängigen Ansprüche.

In einem Aspekt stellt die Erfindung ein Datenübertragungssystem bereit, das personenbezogene und nicht personenbezogene medizinische Daten, vorzugsweise medizinische Daten, überträgt, aufweisend ein ein Formularsystem bereitstellendes erstes Kommunikationsgerät, wobei das Formularsystem dazu eingerichtet ist, Falldaten zu erfassen und wenigstens eine personenbezogene Datenkomponente aus den Falldaten, vorzugsweise automatisch, zu extrahieren und zu wenigstens einem ersten Speicher zu übertragen, wenigstens eine weitere Datenkomponente aus den Falldaten, vorzugsweise automatisch, zu extrahieren und zu wenigstens einem zweiten Speicher zu übertragen, und ein elektronisches Dokument zu generieren und zu einem zweiten Kommunikationsgerät zu senden, und das zweite Kommunikationsgerät, das dazu eingerichtet ist, das elektronische Dokument zu empfangen, und bei Empfang des elektronischen Dokuments, insbesondere auf eine Autorisierung hin, vorzugsweise automatisch, die wenigstens eine personenbezogene Datenkomponente von dem ersten Speicher und die wenigstens eine weitere Datenkomponente von dem zweiten Speicher abzurufen und mit Daten aus dem elektronischen Dokument zu den Falldaten zusammenzuführen und für die Bearbeitung auf dem zweiten Kommunikationsgerät bereit zu stellen.

Das erste Kommunikationsgerät kann dabei eine Schnittstelle aufweisen, über die vor einer Übertragung der Falldatenkomponenten und/oder dem Senden des elektronischen Dokuments eine Autorisierung erfolgt. Die Autorisierung kann durch eine Person erfolgen, auf die sich die personenbezogenen Daten beziehen und/oder durch einen Benutzer, der die Falldaten mittels des Formularsystems erfasst.

Die Autorisierung kann dabei jeweils beispielsweise durch Eingabe eines Codes (Passwort, Schlüssel), durch Einlesen eines Dokuments (Versichertenkarte, Chipkarte, Schlüsselkarte, Smartcard), usw. erfolgen.

Das elektronische Dokument kann einen Verweis auf die wenigstens eine personenbezogene Datenkomponente, die wenigstens eine weitere Datenkomponente und wenigstens einen Schlüssel aufweisen. Der Schlüssel selbst kann, insbesondere mit einem symmetrischen (AES, DES, IDEA, BLOWFISH, TWOFISH, ...) Verfahren, verschlüsselt sein. Das elektronische Dokument kann auch weitere Daten, insbesondere Abrechnungsdaten, aufweisen. Zumindest die wenigstens eine personenbezogene Datenkomponente kann vor der Übertragung, insbesondere mit einem asymmetrischen Verfahren (PGP, GnuPG, Open PGP, RSA, ...), verschlüsselt werden. Der der Schlüssel in dem elektronischen Dokument dient dann zur Entschlüsselung. Der Schlüssel kann dabei insbesondere von einem Schlüsselserver, also einem von einem Computer bereitgestellten Dienst, abgerufen werden. Der Dienst stellt den Schlüssel dann bereit, wenn eine Anfrage mit einem bestimmten, vorzugsweise mit der konsultierenden Partei assoziierten, Identifikator, an den Dienst gestellt wird.

Der erste und der zweite Speicher können räumlich und/oder logisch voneinander getrennt sein. Unter logischer Trennung ist dabei insbesondere die Anordnung in verschiedenen Kommunikationsnetzen, z.B. in verschieden IP-(Sub-)Netzen und/oder verschiedenen physikalischen Netzen, zu verstehen. Die Speicher sind dabei Server, also Computersysteme, die entsprechende Speicherdienste zur Verfügung stellen und entsprechende Kommunikationsschnittstellen aufweisen.

Zumindest der erste Speicher kann dazu eingerichtet sein, die wenigstens eine personenbezogene Datenkomponente zu speichern und nach einer vorbestimmten Zeit und/oder sobald die wenigstens eine personenbezogene Datenkomponente durch das zweite Kommunikationsgerät abgerufen wurde, zu löschen.

Zumindest die wenigstens eine personenbezogene Datenkomponente kann vor Übertragung zu dem ersten Speicher, z.B. durch das Formularsystem, in Datenkomponentenpakete aufgeteilt werden. Die Datenkomponentenpakete können in dem ersten Speicher verschränkt mit anderen Daten abgelegt werden. Die Datenkomponentenpakete können dabei lediglich durch Daten aus dem elektronischen Dokument, insbesondere durch Verweise darauf und/oder durch Angabe ihre Reihenfolge, wieder zu der wenigstens einen personenbezogenen Datenkomponente kombiniert werden.

Nach Bearbeitung der Falldaten können lediglich veränderte nicht personenbezogene Daten zu dem ersten Kommunikationsgerät übermittelt werden. Insbesondere kann diese Übermittlung durch ein Speichern der veränderten nicht personenbezogenen Daten in dem ersten Speicher, bzw. durch eine Veränderung der dort gespeicherten Daten, erfolgen.

Nach Bereitstellung der Falldaten in dem ersten Speicher und dem zweiten Speicher kann eine Benachrichtigung an das zweite Kommunikationsgerät erfolgen. Nach Bereitstellung der bearbeiteten Falldaten kann eine Benachrichtigung des ersten Kommunikationsgeräts erfolgen.

In einem weiteren Aspekt stellt die Erfindung ein Datenübertragungsverfahren für personenbezogene und nicht personenbezogene medizinische Daten bereit, wobei Falldaten mittels eines Formularsystems auf einem ersten Kommunikationsgerät erfasst werden, das wenigstens eine personenbezogene Datenkomponente aus den Falldaten, vorzugsweise automatisch, extrahiert und zu wenigstens einem ersten Speicher überträgt, wenigstens eine weitere Datenkomponente aus den Falldaten, vorzugsweise automatisch, extrahiert und zu wenigstens einem zweiten Speicher überträgt, und ein elektronisches Dokument erzeugt und zu einem zweiten Kommunikationsgerät sendet, und das zweite Kommunikationsgerät das elektronische Dokument empfängt, und bei Empfang des elektronischen Dokuments, insbesondere auf eine Autorisierung hin, vorzugsweise automatisch, die wenigstens eine personenbezogene Datenkomponente von dem ersten Speicher und die wenigstens eine weitere Datenkomponente von dem zweiten Speicher abruft und mit Daten aus dem Elektronischen Dokument zu den Falldaten zusammenführt und für die Bearbeitung auf dem zweiten Kommunikationsgerät bereitstellt.

Die personenbezogenen und die nicht personenbezogenen Daten befinden sich daher während der Übertragung niemals gemeinsam auf einem System/Server, sondern sind stets separiert. Sie werden erst durch die konsultierte Partei wieder zusammengeführt.

Daher sind bei einem erfolgreichen Angriff auf eine Datenkomponente die Informationen aus der jeweils anderen Datenkomponente noch nicht zugänglich.

### Kurze Beschreibung der Figuren

Die Erfindung wird im Weiteren auch mit Bezug auf die Figuren beschrieben:
- Fig. 1: zeigt eine schematische Übersicht über das erfinderische System.
- Fig. 2: zeigt schematisch die Speicherung von personenbezogenen und nicht personenbezogenen Daten.
- Fig. 3: zeigt schematisch ein elektronisches Dokument.
- Fig. 4: veranschaulicht den Löschalgorithmus des Filestores.
- Fig. 5: veranschaulicht die Aufteilung benutzerbezogener Daten nach einem Aspekt der Erfindung.
- Fig. 6: zeigt eine schematische Übersicht über das Gesamtsystem.
- Fig. 7: zeigt ein Ablaufdiagram eines Ausführungsbeispiels des Verfahrens.
- Fig. 8: zeigt einen weiteren schematischen Überblick über das System.

### Detaillierte Beschreibung der Erfindung

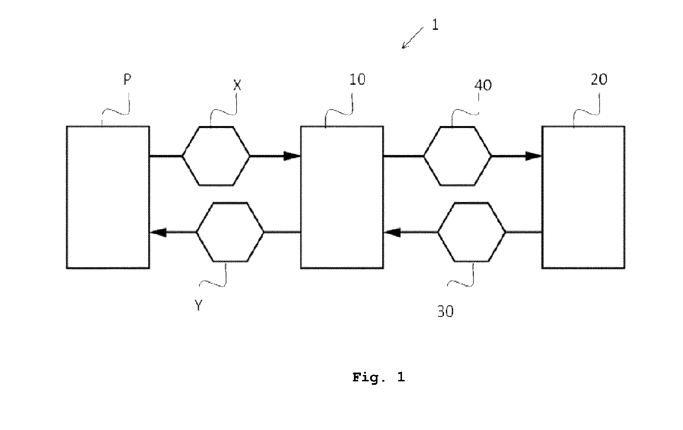

Wie bereits erwähnt, handelt es sich bei dem erfinderischen System um ein Datenkommunikationssystem für den Austausch von Informationen zwischen zwei Parteien, einer konsultierenden, informationseinfordernden Partei, und einer konsultierten, informationsbereitstellenden Partei. Die Informationen beziehen sich dabei auf einen Fall, beispielsweise eine Person oder einen Vorgang. Eine schematische Übersicht über das System 1 ist in **Fig. 1** gezeigt.

In **Fig. 1** ist z.B. ein Fall P dargestellt, über den durch die konsultierende Partei 10 Daten im Schritt X erhoben werden. Die konsultierende Partei 10 erfasst die Falldaten mit einem Formularsystem, z.B. durch einen Benutzer oder automatisiert, und versendet die Falldaten im Schritt 40 an die konsultierte Partei 20. Die konsultierte Partei 20 kann dabei explizit von der konsultierten Partei zur Einsicht in die Falldaten autorisiert werden.

Nach Bearbeitung der Falldaten durch die konsultierte Partei 20 werden die bearbeiteten Daten an die konsultierende Partei 10 im Schritt 30 übermittelt. Diese kann dann das Ergebnis der Konsultation zu dem Fall P weitergeben.

Die Falldaten enthalten dabei nicht personenbezogene Daten und personenbezogene Daten.

Unter nicht personenbezogenen Daten sind insbesondere Daten zu verstehen, die keinen Rückschluss auf den Fall P zulassen. Im Gegensatz dazu erlauben die personenbezogenen Daten die Zuordnung der Falldaten zu dem Fall P. Unter nicht personenbezogenen Daten sind z.B. Anamnesedaten, Blutwerte, Angaben zum Gesundheitszustand, Untersuchungsergebnisse, ... zu verstehen, also insbesondere Daten, die für die konsultierte Partei 20 zur Bearbeitung der Falldaten wichtig sind.

Personenbezogene Daten sind beispielsweise Adressdaten, digitale Dokumente, die persönlich identifizierbare Daten enthalten (z.B. Röntgenbilder, Laborwertberichte, Arztbriefe, usw.) oder die auf andere Art und Weise den Fall P identifizieren können.

Das System 1 besteht prinzipiell aus mehreren Computersystemen, die voneinander unabhängig und getrennt sind. Das System 1 stellt dabei eine separate Übermittlung der personenbezogenen Daten von der konsultierenden zur konsultierten Partei (z.B. Versicherungs-/Versicherten-Nummer, Krankenkasse,...), bereit.

Zur sicheren Übertragung der personenbezogenen und der nicht personenbezogenen Daten geht die Erfindung daher wie folgt vor:
Zunächst erfolgt eine automatische Partitionierung der Falldaten in personenbezogene Daten, die nur vorübergehend gespeichert werden dürfen, und nicht personenbezogene Daten, die beispielsweise auch dauerhaft zum Zwecke einer statistischen Auswertung gespeichert werden dürfen. Dies kann z.B. automatisiert bei dem Erfassen der Falldaten erfolgen, z.B. durch das Formularsystem.

Unter Datenpartitionierung ist dabei eine Aufteilung eben der Falldaten in nicht personenbezogenen und personenbezogenen Daten zu verstehen. Dabei erfolgt die Partitionierung so, dass die eine Komponente der Falldaten keine Referenz zu der anderen Komponente der Falldaten aufweist. Durch Speicherung der Falldatenkomponenten in zwei voneinander unabhängigen Speichern, d.h. auf getrennten, Computersystemen, die z.B. räumlich separiert voneinander angeordnet sind, ist das Wiederherstellen der gesamten Falldaten nur dann möglich, wenn ein Zugang zu beiden Falldatenkomponenten besteht. Zudem sind weitere Informationen nötig, um auf die Falldaten zugreifen zu können.

Dabei werden die nicht personenbezogenen Daten z.B. über ein Formularsystem, das eine Plausibilitätsprüfung durchführen kann, erfasst. Dabei können in dem Formularsystem bereits abhängig von der konsultierten Partei Informationen darüber hinterlegt sein, welche Falldaten von der konsultierten Partei zur Bearbeitung benötigt werden und daher von der konsultierenden Partei zu erfassen sind. Dabei kann das Formularsystem Funktionen bereitstellen, die überprüfen, ob personenbezogene Daten innerhalb der nicht personenbezogenen Daten erfasst werden. So kann das Formularsystem verhindern, dass z.B. Namen oder Versichertennummern angegeben werden, insbesondere in den nicht personenbezogenen Daten, die über die zur Konsultation notwendigen Anamnesedaten hinausgehen.

Durch das Formularsystem werden auch personenbezogene Daten wie beispielsweise Name, Versichertennummer usw. automatisch aus den eingegebenen nicht personenbezogenen Daten entfernt.

Personenbezogene Daten können in dem System 1 vorzugsweise lediglich über Komponentendaten, wie beispielsweise Dateianhänge gespeichert werden. Diese können in verschiedenen Formaten vorliegen wie beispielsweise PDF, Grafikdateien (JPEG, BMP, PNG, TIFF, DICOM), Protokolldaten medizinischer Systeme (XML-Daten, Textdateien, Scans, ...) aber auch Röntgenbilder, MRI-Daten, CT-Daten usw. Da hier nicht ausgeschlossen werden kann, dass diese Daten personenbezogene Angaben enthalten (Arztbrief, Blutwerte, usw. mit z.B. Namen, Versichertennummer, Geburtsdatum, usw.) werden diese Daten nach Erfassung durch das Formularsystem bei Übermittlung der Falldaten sofort getrennt von den nicht personenbezogenen Daten gespeichert.

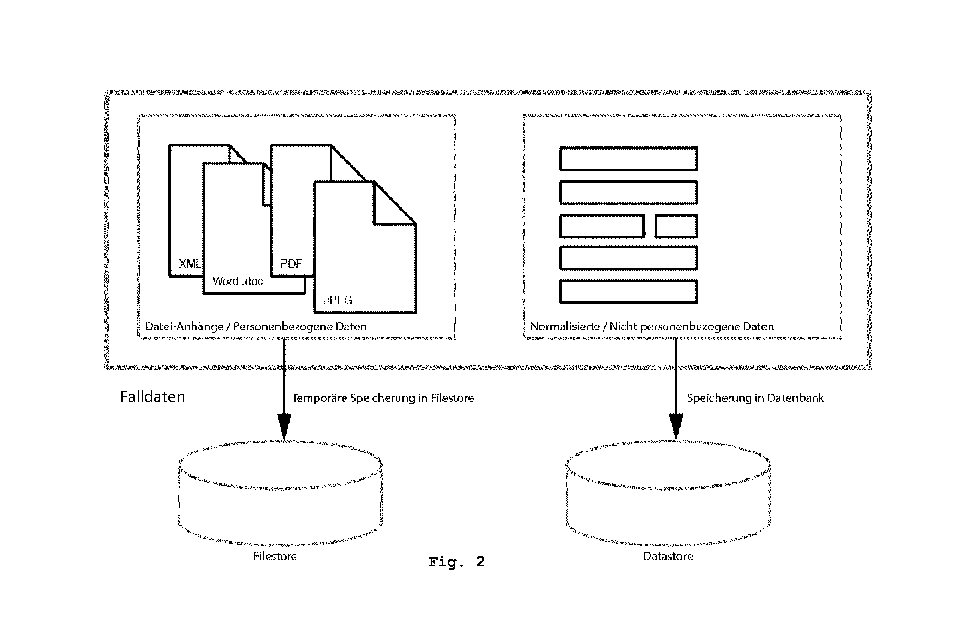

Die personenbezogenen Daten werden dabei auf einem Computersystem gespeichert, das im Folgenden als "Filestore" bezeichnet wird, während die nicht personenbezogenen Daten auf einem Computersystem gespeichert werden, das im Folgenden "Datastore" genannt wird. Dies ist in Fig. 2 gezeigt.

Die Speicherung kann dabei innerhalb jeweils einer Datenbank erfolgen, wobei insbesondere die nicht personenbezogenen Daten von dem Formularsystem in eine Datenbank des Datastore gespeichert werden können. Die personenbezogenen Daten können in dem Filestore auch über ein Dateisystem gespeichert werden, z.B. ein Protokoll (SMB, NFS, FTP, SFTP, SCP, ...), das den Zugriff auf Dateien über ein Netzwerk ermöglicht. Dabei kann es durch das System 1 ermöglicht werden, dass beide Parteien auf die im Filestore abgelegten Daten, so zugreifen, als ob sie lokal abgespeichert wären. Die Daten aus dem Datastore können z.B. mit einem Formularsystem auf Seite der konsultierten Partei 20 abgefragt werden.

Zudem werden neben den nicht personenbezogenen Daten und den personenbezogenen Daten weitere Daten von der konsultierenden Partei 10 zu der konsultierten Partei 20 übermittelt, die im Folgenden als elektronisches Dokument 50 bezeichnet werden.

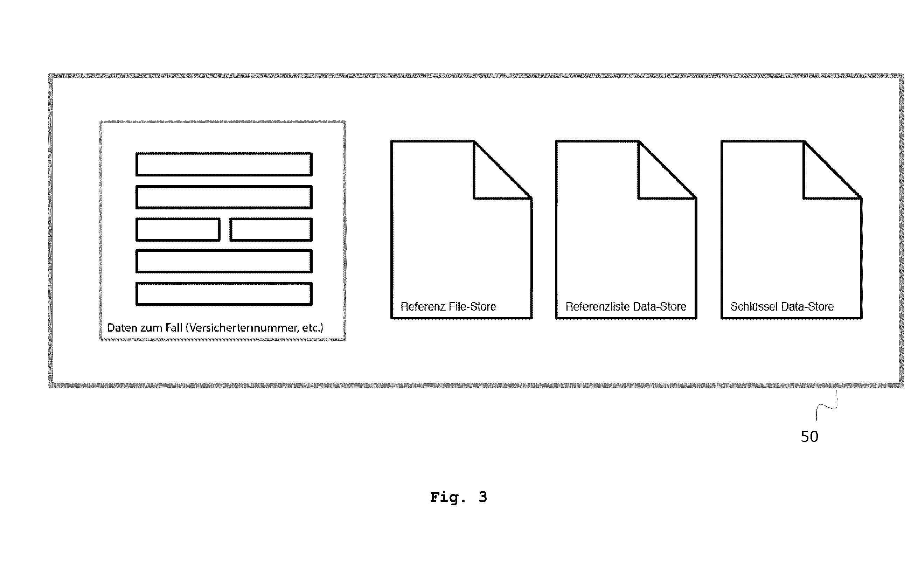

Insbesondere ist darunter ein elektronisches Dokument 50 zu verstehen, das mit einem Verschlüsselungsverfahren, beispielsweise einem Privat-Public Key Verfahren verschlüsselt ist. Eine schematische Darstellung des elektronischen Dokumentes 50 ist in **Fig. 3** gezeigt.

Das elektronische Dokument 50 kann beispielsweise eine elektronische Überweisung (E-Referral) sein. Dabei enthält das elektronische Dokument 50 beispielsweise Abrechnungsdaten wie Versichertennummer, Name, Geburtsdatum, usw., einen Schlüssel zur Entschlüsselung der Falldaten, so wie Verweise auf die personenbezogenen Daten im Filestore und die nicht personenbezogenen Daten im Datastore.

Nach Erfassung aller relevanten Falldaten durch das Formularsystem wird das elektronische Dokument 50 ebenfalls durch das System 1 erzeugt und an die konsultierte Partei 20 zugestellt. Dies kann z.B. auch mittels E-Mail erfolgen. Dadurch ist sichergestellt, dass lediglich die konsultierte Partei 20 und die konsultierende Partei 10 Zugriff auf Falldaten erhalten.

Mit Übermittlung des elektronischen Dokuments 50 erfolgt also im Wesentlichen die Autorisierung der konsultierten Partei 20, also die Berechtigung die Falldaten einsehen und bearbeiten zu können. Die konsultierte Partei 20 erhält durch das elektronische Dokument 50 beispielsweise Zugriff auf relevante Daten, die zum Beispiel in einem kassenärztlichen System zur Abrechnung von Leistungen notwendig sind. Dies können, wie bereits erwähnt, beispielsweise die Versichertennummer, Name, Vorname, Geburtsdatum, Adresse, usw. sein.

Das elektronische Dokument 50 ist insbesondere Fallbezogen, wird also immer für einen Fall erzeugt, bzw. für eine (einzige) Beratung. Das elektronische Dokument 50 enthält einen Verweis (Referenz, Link) auf die nicht personenbezogenen Daten der Falldaten, die im Datastore gespeichert sind, bzw. die entsprechende Datenkomponente, die zu dem Fall P gehört, auf den sich das elektronische Dokument 50 bezieht. Weiter ist ein Verweis auf die personenbezogenen Daten der Falldaten, die in dem Filestore gespeichert sind, in dem elektronischen Dokument 50 enthalten, ebenfalls abhängig von dem Fall P.

Das elektronische Dokument 50 wird beispielsweise automatisch nach Erfassung aller relevanten Daten durch das Formularsystem erzeugt und gesichert, beispielsweise durch ein Verschlüsselungsverfahren verschlüsselt, jeweils nur an die zu autorisierende, also die konsultierte Partei 20, übermittelt. Nur diese Partei kann das elektronische Dokument entschlüsseln. Das elektronische Dokument 50 kann wenigstens einen Schlüssel enthalten, der wiederum, beispielsweise mit einem symmetrischen Verfahren verschlüsselt sein kann, und der genau für das erzeugte elektronische Dokument 50 gültig ist, mit dem er übermittelt wird. Mit Hilfe dieses wenigstens einen Schlüssels kann dann auf die Daten, auf die in dem elektronischen Dokument verwiesen wird, zugegriffen werden. Dabei ist es auch möglich, für verschiedene Datenteile der Datenkomponenten, bzw. für die verschiedenen Datenkomponenten selbst, verschiedene Schlüssel vorzusehen. Beispielsweise kann ein Schlüssel für alle oder einen Teil der personenbezogenen Daten, bzw. der nicht personenbezogenen Daten, vorgesehen sein.

Zur Übertragung des elektronischen Dokumentes 50 ist vorzugsweise eine gesicherte Übertragung vorgesehen. Beispielsweise kann vorgesehen sein, das elektronische Dokument direkt durch Datenfernübertragung von der konsultierenden Partei zu der konsultierten Partei über das Telefonnetz zu übertragen (z.B. per Fax mit maschinenlesbarem Code (Barcode, QR-Code, Matrix-Code, ...).

Alternativ oder zusätzlich kann vorgesehen sein das elektronische Dokument insgesamt verschlüsselt, aber über öffentliche Netze, wie beispielsweise das Internet, zu übermitteln. Auch die Übertragung mittels verschlüsselter Kanäle, die beispielsweise durch TLS (Transport Layer Security) und/der SSL (Secure Socket Layer) gesichert sind, ist vorgesehen.

Insgesamt können selbstverständlich auch die nicht personenbezogenen und die personenbezogenen Daten über gesicherte Verbindungen zu den jeweiligen Speichern übertragen werden.

Es ist jedoch Ziel der Erfindung, insbesondere personenbezogene Daten nicht dauerhaft auf einem System wie beispielsweise einem Server zu speichern. Da aber eine asynchrone Übertragung zwischen der konsultierenden Partei 10 und der konsultierten Partei 20 ermöglich werden soll, also beide Parteien nicht dazu gezwungen sein sollen, zur gleichen Zeit für die Kommunikation bereit zu stehen, ist ein Speichern auf einem verteilten System vorgesehen.

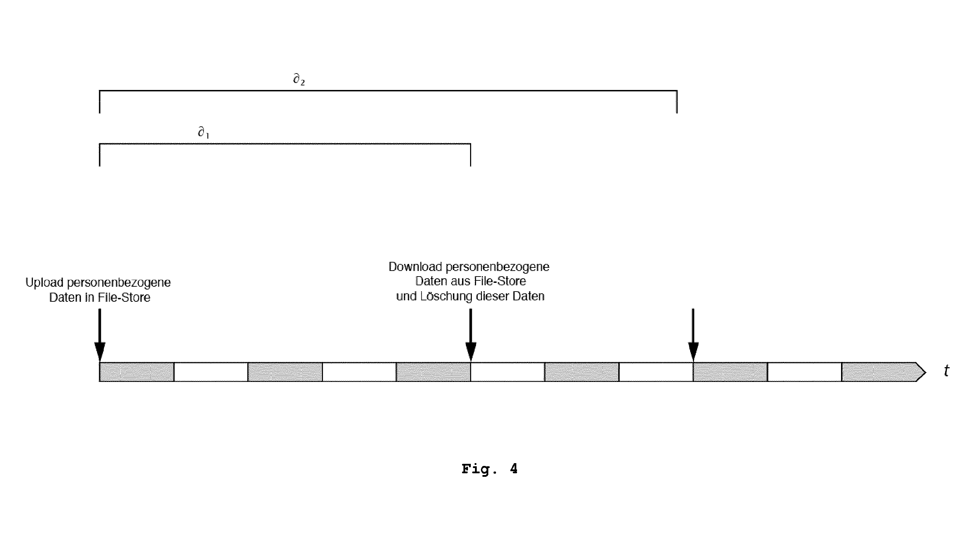

Die konsultierende Partei 10 lädt also die personenbezogenen und die nicht personenbezogenen Daten auf jeweils einen Speicher (Upload) während die konsultierte Partei 20 nach Erhalt des elektronischen Dokuments die personenbezogenen und die nicht personenbezogenen Daten von dem jeweiligen Speicher herunterlädt (Download). Um die Möglichkeit für einen Angriff auf insbesondere die personenbezogenen Daten zu reduzieren, werden zumindest die personenbezogenen Daten nach dem Download von dem Speicherort durch das System 1 gelöscht. Dies ist in **Fig. 4** veranschaulicht.

In **Fig. 4** ist gezeigt, dass die personenbezogenen Daten nach der Zeit δ₁, d.h. nach Abruf der Daten durch die konsultierte Partei 20, gelöscht werden.

Wenigstens die personenbezogenen Daten werden nach einer vorbestimmten Zeit δ₂, beginnend mit dem Ende des Uploads, von dem Speicherort gelöscht. Sollte ein Download nach dieser vorbestimmten Zeit δ₂ nicht durch die konsultierte Partei 20 erfolgt sein, erhält die konsultierte Partei 20 die Möglichkeit, die konsultierende Partei 10 zu benachrichtigen, um den Upload erneut zu starten, also die entsprechenden Daten erneut zur Verfügung zu stellen. Nach Ablauf der Zeit δ₂ kann das System 1 dann die konsultierte Partei 20 darüber informieren, dass die vorbestimmte Zeit δ₂ abgelaufen ist und automatisch die Möglichkeit bereitstellen, die Informationen erneut anzufordern.

Als weitere Sicherheitsfunktion ist vorgesehen, die in dem Filestore, also die Datenkomponente mit personenbezogenen Daten, zu speichernden Daten in Pakete aufzuteilen, und diese getrennt voneinander in dem Filestore abzulegen. Dabei erfolgt die Aufteilung der personenbezogenen Daten auf Seite der konsultierenden Partei 10 und die Pakete werden dann zu dem Filestore übertragen.

In dem Filestore werden die Pakete dann in zufälliger Reihenfolge und vermischt (verschränkt) mit anderen Daten, beispielsweise mit Daten anderer Fälle, gespeichert. Selbst wenn ein Angreifer also Zugriff auf den Filestore erhalten sollte, kann er mit den dort aufgefundenen Datenfragmenten zunächst nichts anfangen. Lediglich das elektronische Dokument 50 enthält in diesem Fall Verweise auf die einzelnen Datenpakete der jeweiligen Datenkomponente. Lediglich durch das elektronische Dokument 50 ist die konsultierte Partei 20 in der Lage, die personenbezogenen Daten wieder herzustellen. Selbstverständlich ist es auch möglich, die nicht personenbezogenen Daten auf diese Weise zu speichern.

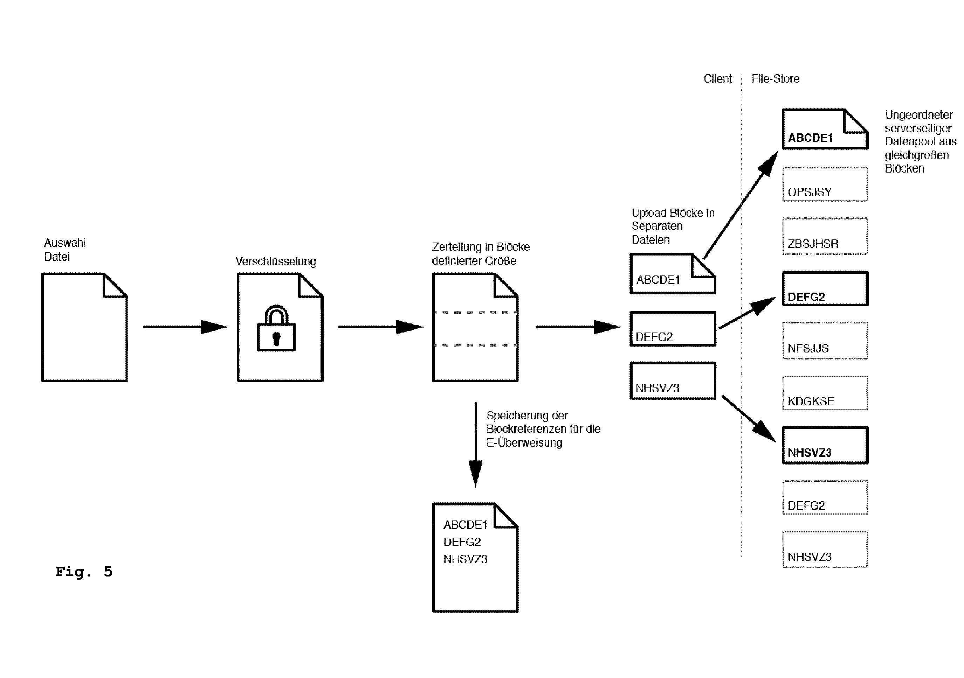

Eine schematische Darstellung dieses Verfahrens ist in **Fig. 5** dargestellt. Hier werden die personenbezogenen Daten beispielsweise in Daten-Blöcke gleicher Größe aufgeteilt, die dann als einzelne Dateien auf den Filestore gespeichert werden. Der "Datenpool" auf dem Filestore enthält dann viele Dateien, die ohne Verbindung zueinander abgelegt sind. Lediglich die dargestellte Überweisung enthält die Verweise auf die benötigten Dateien.

Als weitere Bestandteile kann das System 1 einen Schlüsselserver/-speicher (Keystore) aufweisen, der beispielsweise die öffentlichen Schlüssel der an dem System beteiligten Parteien verwaltet. Der Schlüsselserver kann dabei auch die Nutzerregistrierung und die Freischaltung von Nutzern verwalten. Zudem ist der Schlüsselserver in der Lage, neue Schlüssel zu generieren und neue öffentliche Schlüssel zu speichern. Diese öffentlichen Schlüssel werden dann für die beteiligten Parteien bereitgestellt.

Die bereits erwähnte Erfassung, Verwaltung und Speicherung von Falldaten, die durch das Formularsystem gestützt erfolgen, sowie das Ver- und Entschlüsseln von Falldaten und die Generierung des elektronischen Dokumentes 50 erfolgt vorzugsweise auf einem Kommunikationsgerät. Dies kann beispielsweise durch eine Anwendung auf einem Computer oder einem mobilen Kommunikationsendgerät (Handy, Smartphone, Laptop, Tablet, ...) erfolgen.

Der Datastore, also der Speicher der nicht personenbezogenen Daten, verwaltet diese und kann Funktionen zur statistischen Auswertung bereit stellen. Der Filestore, also der Speicher für die Zwischenspeicherung der personenbezogenen Daten, enthält Mechanismen, um die jeweiligen Daten nach Zeitablauf, nach dem einmaligen Herunterladen (Download) der Daten oder aufgrund anderer Parameter zu löschen.

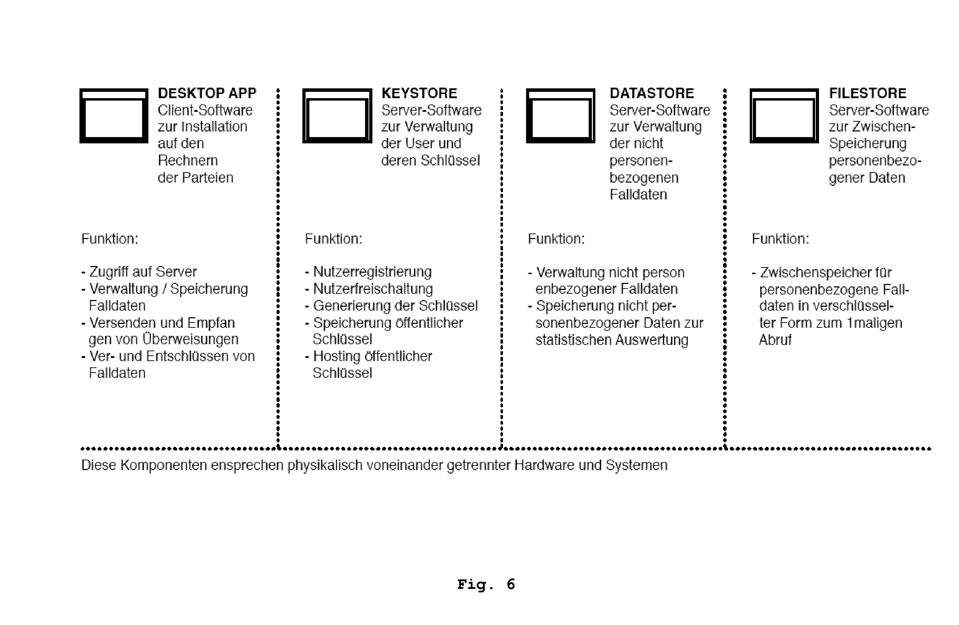

Eine schematische Übersicht des Gesamtsystems ist in **Fig. 6** dargestellt. Es ist klar, dass sich das beschriebene System und Verfahren auch auf andere Bereiche anwenden lässt, in denen sensible und weniger sensible Daten von einer Quelle zu einem Empfänger zu übertragen sind.

Somit kann die Erfindung allgemein dann eingesetzt werden, wenn eine Aufteilung von Daten in wenigstens zwei Komponenten möglich ist. Werden Daten in mehrere Datenkomponenten aufgeteilt, können diese dann zu mehreren Speichern übertragen werden. Ein Einsatz in Verwaltungsorganisationen ist daher ebenfalls vorgesehen.

Auch ist zu verstehen, dass die Übertragung der Daten in die umgekehrte Richtung ebenfalls möglich ist, und hierbei lediglich die Rollen der Parteien, bzw. die von Quelle und Empfänger, vertauscht sind. In diesem Fall kann vorgesehen sein, dass die in "Vorwärtsrichtung" übertagenen Falldaten auf Seite der konsultierten Partei 20 ebenfalls durch ein Formularsystem dargestellt werden, und dass über das Formularsystem dann zusätzliche Daten, z.B. Text und/oder Dateien, den Falldaten hinzugefügt werden können. Diese veränderten Falldaten können dann analog zu der beschriebenen Übermittlung in "Rückwärtsrichtung" zurück übermittelt werden, also z.B. zu der konsultierenden Partei 10.

Auch ist es möglich, dass die Daten von der konsultierten Partei 20 an eine weitere Partei mittels des Systems/Verfahren übermittelt werden können, wobei die Falldaten bei jeder Partei verändert werden, zumindest Daten hinzugefügt werden können.

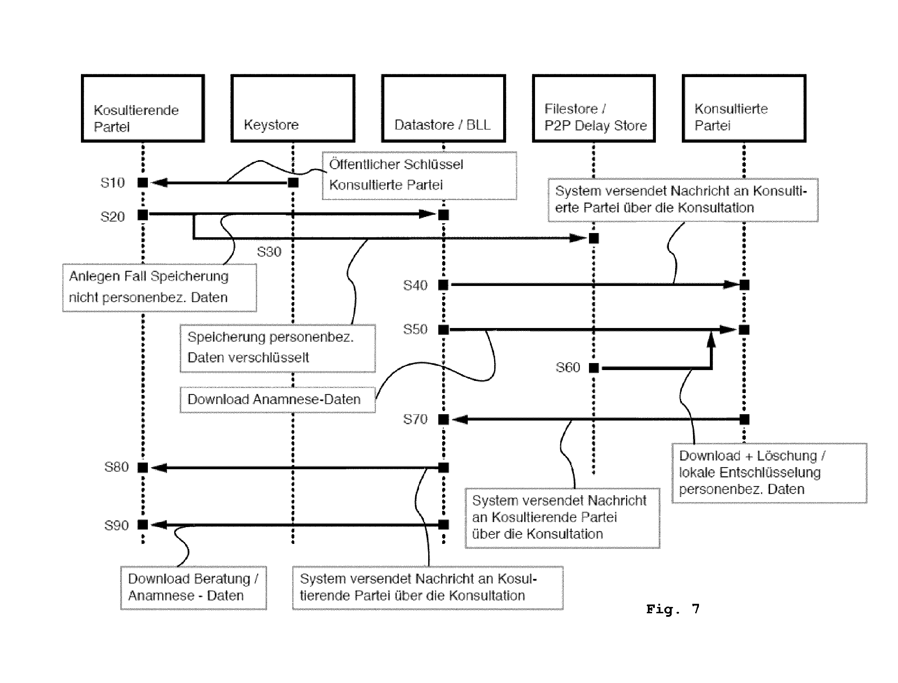

Ein beispielhafter schematischer Ablauf des Verfahrens ist in **Fig. 7** dargestellt. Zunächst ruft hier die konsultierende Partei 10 in Schritt S10 einen öffentlichen Schlüssel der konsultierten Partei 20 von einem Schlüsselserver ab.

Die Falldaten werden dann in den Schritten S20 und S30 sowohl auf dem Filestore als auch auf dem Datastore angelegt und/oder gespeichert. Anschließend wird die konsultierte Partei 20 von dem System 1 in Schritt S40 benachrichtigt, dass Falldaten zur Beratung vorliegen.

In den Schritten S50 und S60 lädt dann die konsultierte Partei 20 die Daten von dem Filestore und dem Datastore, wobei die Daten auf dem Filestore nach dem Herunterladen in Schritt S70 automatisch gelöscht werden.

In dem in **Fig. 7** dargestellten Ausführungsbeispiel lädt nun im Schritt S70 die konsultierte Partei 20 lediglich veränderte nicht personenbezogene Daten auf den Datastore, bzw. verändert die dort gespeicherten Daten z.B. durch Hinzufügen von Informationen. Dies kann durch ein auf Seiten der konsultierten Partei 20 bereitgestelltes Formularsystem erfolgen.

In Schritt S80 in informiert das System 1 die konsultierende Partei 10 darüber, dass die Falldaten von der konsultierten Partei 20 verändert wurden, insbesondere, dass der Fall bearbeitet wurde.

Die veränderten Falldaten, hier lediglich die nicht personenbezogenen Daten, werden dann durch die konsultierende Partei 10 in Schritt S90 heruntergeladen.

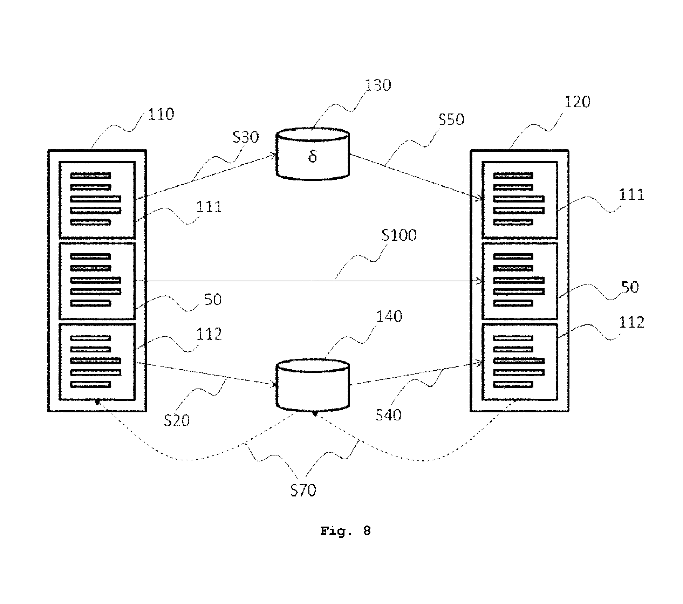

**Fig. 8** zeigt eine weitere schematische Ansicht des Systems 1, das ebenfalls teilweise die Schritte veranschaulicht, die in **Fig. 7** gezeigt sind.

Darin sind die auf Seite der konsultierenden Partei 10 erfassten Falldaten 110 gezeigt, die aus der personenbezogenen Komponente 111 und der nicht personenbezogenen Komponente 112 sowie dem elektronischen Dokument 50 gebildet werden. Der erste Speicher 130 stellt den Filestore dar, der die personenbezogenen Daten, wie oben beschrieben, nach der Zeit δ₁ oder δ₂ löscht. Der zweite Speicher 140 bildet den Datastore für die nichtpersonenbezogenen Daten. Auf Seite der konsultierten Partei 20 werden die Falldaten dann aus den Speichern 130, 140 mit den Daten des elektronischen Dokuments wieder zu den Falldaten 120 zusammengestellt. Die Falldaten 120 bestehen dabei zumindest aus den personenbezogenen Daten 111 aus dem ersten Speicher 130, aus den nicht personenbezogenen Daten 112 aus dem zweiten Speicher 140 sowie Daten aus dem elektronischen Dokument 50. Die Falldaten 110 und Falldaten 120 entsprechen sich dabei. Die Bearbeitung der nicht personenbezogenen Daten ist wiederum mit dem Schritt S70 gezeigt. Die direkte Übertragung des elektronischen Dokuments 50 ist als Schritt S100 dargestellt.

## Patentansprüche

1. Datenübertragungssystem, das personenbezogene und nicht personenbezogene medizinische Daten überträgt, aufweisend:
a. ein ein Formularsystem bereitstellendes erstes Kommunikationsgerät, wobei das Formularsystem dazu eingerichtet ist, Falldaten (110) zu erfassen und
i. wenigstens eine personenbezogene Datenkomponente (111) aus den Falldaten (110) zu extrahieren und zu wenigstens einem ersten Speicher (130) zu übertragen,
ii. wenigstens eine weitere Datenkomponente (112) aus den Falldaten (110) zu extrahieren und zu wenigstens einem zweiten Speicher (140) zu übertragen, und
iii. ein elektronisches Dokument (50) zu generieren und zu einem zweiten Kommunikationsgerät zu senden, und
b. das zweite Kommunikationsgerät, das dazu eingerichtet ist, das elektronische Dokument (50) zu empfangen, und nach Empfang des elektronischen Dokuments (50) die wenigstens eine personenbezogene Datenkomponente (111) von dem ersten Speicher (130) und die wenigstens eine weitere Datenkomponente (112) von dem zweiten Speicher (140) abzurufen und mit Daten aus dem elektronischen Dokument (50) zu den Falldaten (120) zusammenzuführen und für die Bearbeitung auf dem zweiten Kommunikationsgerät bereit zu stellen.

2. Datenübertragungssystem nach Anspruch 1, wobei das erste Kommunikationsgerät eine Schnittstelle aufweist, über die vor einer Übertragung der Falldatenkomponenten (111, 112) und/oder dem Senden des elektronischen Dokuments (50) eine Autorisierung erfolgt, wobei die Autorisierung durch eine Person erfolgt, auf die sich die personenbezogenen Datenkomponente (111) bezieht und/oder durch einen Benutzer, der die Falldaten (110) mittels des Formularsystems erfasst.

3. Datenübertragungssystem nach Anspruch 1 oder 2, wobei das elektronische Dokument (50) einen Verweis auf die wenigstens eine personenbezogene Datenkomponente (111), die wenigstens eine weitere Datenkomponente (112) und wenigstens einen Schlüssel aufweist, wobei der Schlüssel selbst verschlüsselt ist, und wobei das elektronische Dokument (50) weitere Daten aufweist.

4. Datenübertragungssystem nach einem der vorigen Ansprüche, wobei zumindest die wenigstens eine personenbezogene Datenkomponente (111) vor der Übertragung, insbesondere mit einem asymmetrischen Verfahren, verschlüsselt wird, und wobei der Schlüssel in dem elektronischen Dokument (50) zur Entschlüsselung dient.

5. Datenübertragungssystem nach einem der vorigen Ansprüche, wobei der erste Speicher (130) und der zweite Speicher (140) räumlich und/oder logisch voneinander getrennt sind, beispielweise in verschiedenen Kommunikationsnetzen angeordnet sind.

6. Datenübertragungssystem nach einem der vorigen Ansprüche, wobei zumindest der erste Speicher (130) dazu eingerichtet ist, die wenigstens eine personenbezogene Datenkomponente (111) zu speichern und nach einer vorbestimmten Zeit (δ₁) und/oder sobald die wenigstens eine personenbezogene Datenkomponente durch das zweite Kommunikationsgerät abgerufen wurde (δ₂), zu löschen.

7. Datenübertragungssystem nach einem der vorigen Ansprüche, wobei zumindest die wenigstens eine personenbezogene Datenkomponente (111) vor Übertragung zu dem ersten Speicher (130), z.B. durch das Formularsystem, in Datenkomponentenpakete aufgeteilt wird, wobei die Datenkomponentenpakete in dem ersten Speicher (130) verschränkt mit anderen Daten abgelegt werden, und wobei die Datenkomponentenpakete lediglich durch Daten aus dem elektronischen Dokument (50), insbesondere durch Verweise darauf und/oder eine Angabe ihre Reihenfolge, wieder zu der wenigstens einen personenbezogenen Datenkomponente (111) kombinierbar sind.

8. Datenübertragungsverfahren für die Übertragung personenbezogener und nicht personenbezogener medizinischer Daten,
a. wobei Falldaten (110) mittels eines Formularsystems auf einem ersten Kommunikationsgerät erfasst werden,
i. das wenigstens eine personenbezogene Datenkomponente (110) aus den Falldaten extrahiert und zu wenigstens einem ersten Speicher (130) überträgt,
ii. wenigstens eine weitere Datenkomponente (112) aus den Falldaten extrahiert und zu wenigstens einem zweiten Speicher (140) überträgt, und
iii. ein elektronisches Dokument (50) erzeugt und zu einem zweiten Kommunikationsgerät sendet, und
b. das zweite Kommunikationsgerät das elektronische Dokument (50) empfängt, und nach Empfang des elektronischen Dokuments die wenigstens eine personenbezogene Datenkomponente (111) von dem ersten Speicher (130) und die wenigstens eine weitere Datenkomponente (112) von dem zweiten Speicher (140) abruft und mit Daten aus dem Elektronischen Dokument (50) zu den Falldaten zusammenzuführt und für die Bearbeitung zugänglich macht.

9. Datenübertragungsverfahren nach Anspruch 8, wobei vor einer Übertragung der Falldatenkomponenten (111, 112) und/oder dem Senden des elektronischen Dokuments (50) eine Autorisierung über eine Schnittstelle des ersten Kommunikationsgeräts erfolgt, wobei die Autorisierung durch eine Person erfolgt, auf die sich die Personenbezogenen Datenkomponente (111) bezieht und/oder durch eine Partei, die die Falldaten (110) mittels des Formularsystems erfasst.

10. Datenübertragungsverfahren nach Anspruch 8 oder 9, wobei das elektronische Dokument (50) einen Verweis auf die wenigstens eine personenbezogene Datenkomponente (111), die wenigstens eine weitere Datenkomponente (112) und wenigstens einen Schlüssel aufweist, wobei der Schlüssel selbst verschlüsselt ist, und wobei das elektronische Dokument (50) weitere Daten aufweist.

11. Datenübertragungsverfahren nach einem der vorigen Ansprüche, wobei zumindest die wenigstens eine personenbezogene Datenkomponente (111) vor der Übertragung, insbesondere mit einem asymmetrischen Verfahren, verschlüsselt wird, und wobei der Schlüssel in dem elektronischen Dokument (50) zur Entschlüsselung dient.

12. Datenübertragungsverfahren nach einem der vorigen Ansprüche, wobei der erste Speicher (130) und der zweite Speicher (140) räumlich und/oder logisch voneinander getrennt sind, beispielweise in verschiedenen Kommunikationsnetzen angeordnet sind.

13. Datenübertragungsverfahren nach einem der vorigen Ansprüche, wobei zumindest der erste Speicher (130) die wenigstens eine personenbezogene Datenkomponente (111) speichert und nach einer vorbestimmten Zeit (δ₁) und/oder sobald die wenigstens eine personenbezogene Datenkomponente durch das zweite Kommunikationsgerät abgerufen wurde (δ₂), löscht.

14. Datenübertragungsverfahren nach einem der vorigen Ansprüche, wobei zumindest die wenigstens eine personenbezogene Datenkomponente (111) vor Übertragung zu dem ersten Speicher (130), z.B. durch das Formularsystem, in Datenkomponentenpakete aufgeteilt wird, wobei die Datenkomponentenpakete in dem ersten Speicher (130) verschränkt mit anderen Daten abgelegt werden, und wobei die Datenkomponentenpakete lediglich durch Daten aus dem elektronischen Dokument (50), insbesondere durch Verweise darauf und/oder eine Angabe ihre Reihenfolge, wieder zu der wenigstens einen personenbezogenen Datenkomponente (111) kombinierbar sind.

15. Datenübertragungsverfahren nach einem der vorigen Ansprüche, wobei nach Bearbeitung der Falldaten lediglich veränderte nicht personenbezogene Daten zu dem ersten Kommunikationsgerät übermittelt werden, insbesondere durch ein Speichern in dem ersten Speicher (130), und wobei nach Bereitstellung der Falldaten in dem ersten Speicher (130) und dem zweiten Speicher (140) eine Benachrichtigung an das zweite Kommunikationsgerät erfolgt, und wobei nach Bereitstellung der bearbeiteten Falldaten eine Benachrichtigung des ersten Kommunikationsgeräts erfolgt.
